(19) Europäisches Patentamt
European Patent Office
Office européen des brevets

(11) Publication number: **0 067 711**
**B1**

# (12) EUROPEAN PATENT SPECIFICATION

(45) Date of publication of patent specification: **01.10.86**

(51) Int. Cl.⁴: **C 07 D 217/20,** A 61 K 31/47

(21) Application number: **82303100.0**

(22) Date of filing: **15.06.82**

## (54) New 1-benzyl-3,4-dihydro-isoquinoline derivative.

(30) Priority: **16.06.81 HU 176881**

(43) Date of publication of application:
**22.12.82 Bulletin 82/51**

(45) Publication of the grant of the patent:
**01.10.86 Bulletin 86/40**

(84) Designated Contracting States:
**AT BE CH DE FR GB IT LI NL SE**

(56) References cited:
**NL-A-7 804 202**

**EUROPEAN JOURNAL OF DRUG METABOLISM AND PHARMACOKINETICS, vol. 5, no. 2, 1980, pages 69-74, Geneva (CH); Z. VARGAY et al.: "Qualitative and quantitative determination of drotaverine metabolites in rat bile"**

**CHEMICAL ABSTRACTS, vol. 96, no. 7, 15th February 1982, p. 12, no. 45814s, Columbus, Ohio (US); I.SZATMARY et al.: "Pharmacokinetics and metabolism of depogen in rats"**

(73) Proprietor: **CHINOIN Gyogyszer és Vegyészeti Termékek Gyára RT.**
**To utca 1-5**
**H-1045 Budapest IV (HU)**

(72) Inventor: **Szantay, Csaba**
**8 Zsombolyai u**
**H-1113 Budapest (HU)**
Inventor: **Kalaus, Gyorgy**
**3/E Bartok B ut**
**H-1225 Budapest (HU)**
Inventor: **David, Agoston**
**12 Batthyany u**
**H-1015 Budapest (HU)**
Inventor: **Gyori, Peter**
**32 Gyakorlo u**
**H-1106 Budapest (HU)**
Inventor: **Szekeres, Laszlo**
**2 Kazinczy u**
**H-6720 Szeged (HU)**
Inventor: **Papp, Gyula**
**37-39 Becsi krt**
**H-6722 Szeged (HU)**
Inventor: **Udvary, Eva**
**2/A Olajos u.**
**H-6723 Szeged (HU)**

**EP 0 067 711 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

(74) Representative: **Skailes, Humphrey John et al**
**Frank B. Dehn & Co. Imperial House 15-19**
**Kingsway**
**London WC2B 6UZ (GB)**

**Description**

This invention relates to a new 1-benzyl-3,4-dihydro-isoquinoline derivative, a process for the preparation thereof and pharmaceutical compositions containing the same.

According to a feature of the present invention there is provided the new 6,7-diethoxy-1-(4-ethoxy-3-hydroxy-benzyl)-3,4-dihydro-isoquinoline of the Formula I

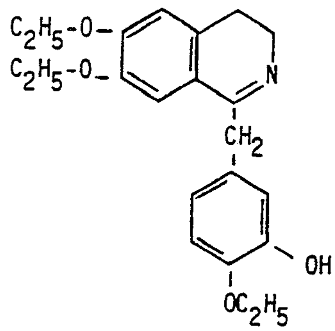

(I)

and physiologically acceptable salts thereof.

The new compound of the Formula I and salts thereof possess useful physiological properties and exhibit particularly useful effects on the circulation.

The physiologically acceptable salts of the Formula I may be formed with inorganic or organic acids e.g. hydrochloric acid, hydrobromic acid, sulfuric acid, phosphoric acid, acetic acid, oxalic acid, tartaric acid, succinic acid, citric acid, maleic acid, salicylic acid or benzoic acid. The salts may be neutral (e.g. sulfates or phosphates) or acidic (e.g. hydrogen sulfates, hydrogen phosphates) salts.

According to a further feature of the present invention there is provided a process for the preparation of the compound of the Formula I and physiologically acceptable salts thereof which comprises hydrolysing a compound of the general Formula VI

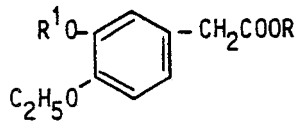

(VI),

(wherein $R^1$ is a group removable by hydrogenolysis, preferably a benzyl group and R is an alkyl group having 1—6 carbon atoms), reacting the compound of the general Formula V

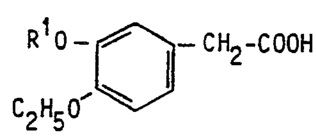

(V),

thus obtained (wherein $R^1$ is as stated above) with an amine of the Formula IV

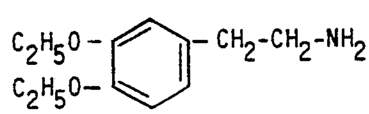

(IV)

or a salt thereof in the presence of a catalyst, subjecting the amide of the general Formula III

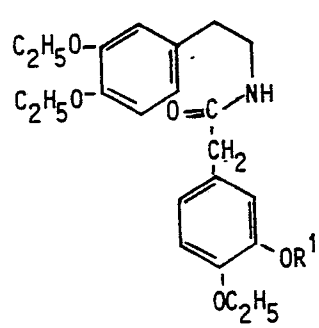

(III) ,

thus obtained (wherein $R^1$ is as stated above) to ring-closure and subjecting the compound of the general Formula II

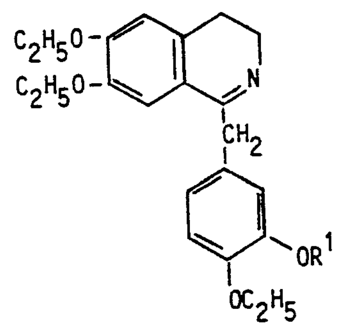

(II) ,

(wherein $R^1$ is as stated above) or a salt thereof thus obtained to hydrogenolysis and if desired converting the compound of the Formula I thus obtained into a physiologically acceptable salt thereof or setting free the same from its salt.

The term "alkyl group having 1—6 carbon atoms" relates to straight or branched chained saturated aliphatic hydrocarbon groups having 1—6 carbon atoms (e.g. methyl, ethyl, *n*-propyl, isopropyl, *n*-butyl etc.). $R^1$ may stand for any suitable group which can be removed by hydrogenolysis and preferably means benzyl.

In the first step of the process of the present invention a compound of the general Formula VI is hydrolysed preferably with an alkali metal hydroxide. On acidifying the reaction mixture with a mineral acid a compound of the general Formula V is obtained and the precipitated solid product can be isolated preferably by filtration.

The reaction of the compounds of the general Formulae IV and V is preferably carried out in a solvent (e.g. benzene, toluene or xylene) in the presence of a catalyst capable of delivering protons. As catalyst capable of delivering protons mineral acids, sulfonic acids or cation-exchanging resins can be used. The reaction can be carried out preferably at the boiling point of the mixture and the water formed in the reaction is removed by azeotropic distillation. The reaction having been completed the reaction mixture is cooled and the precipitated acid amide of the general Formula III is isolated by filtration.

The cyclisation of the compounds of the general Formula III can be carried out in the presence of a condensing agent (preferably phosphoryl chloride, phosphorous pentachloride, thionyl chloride or polyphosphoric acid or a mixture thereof) in the presence or absence of an organic solvent. As solvent e.g. aromatic hydrocarbons or acetonitrile can be used. Cyclisation can be carried out in a broad temperature range.

The compounds of the general Formula II thus obtained are subjected to hydrogenolysis. The reaction can be preferably accomplished by means of catalytic hydrogenation and can be advantageously effected in the presence of an acid. The catalytic hydrogenation of the compounds of the general Formula II can be carried out in a solvent (e.g. lower aliphatic alcohols) in the presence of a heterogenous catalyst (e.g. Raney-nickel, platin or palladium charcoal). Hydrogenation can be carried out under atmospheric or higher pressure.

The compound of the Formula I can be isolated from the reaction mixture by known methods. The compound of the Formula I can be purified by crystallization from suitable lower aliphatic alcohols but a methanol-ether mixture or ethyl acetate can also be used as crystallizing solvent.

The compound of the Formula I can be converted into its salts formed with physiologically acceptable inorganic or organic acids (e.g. aromatic or aliphatic mono- or dicarboxylic acids, hydroxy acids, alkyl sulfonic acids, aryl sulfonic acids, amino acids or mineral acids) by methods known per se. The compound of the Formula I can also be set free from its salts by known methods.

The preparation of the starting material of the Formula VI is known from prior art (J. Het. Chem. *7*, 339—343 (1970)].

The 6,7-diethoxy-1-(3-ethoxy-4-hydroxy-benzyl)-3,4-dihydro-isoquinoline being structurally similar to the compound of the Formula I can also be prepared by the process of the present invention and is known from prior art. [European Journal of Drug Metabolism and Pharmacokinetics *5* (2) 69—74 (1980)].

The compound of the Formula I exhibits useful physiological and particularly cardiovascular effects. The anti-angina activity is determined by measuring the inhibition of the acute coronary insufficiency (which corresponds to the rise of the T-wave on the electrocardiogram) induced by vasopressine (2 IU/kg i.v.) on pentobarbital anaesthetized (45 mg/kg i.p.) rats belonging to the CFY strain (method of Papp and Szekeres: Arch. Int. Pharmacodyn. *160*, 147 /1966/). The antiarrhythmial effect is determined on chloralose-urethane anaesthetized cats [500/300 mg/kg i.p.] by measuring the changes of the electric fibrillation threshold of the auricle and ventricle respectively [method of Szekeres, Méhes and Papp: Brit. J. Pharmacol. *17*, 167 (1961) and that of Szekeres and Papp: Hdb. exp. Pharmacol. *16/3*, 131 (1975)

respectively]. The general effects exerted on the haemodynamic and cardiovascular system are tested on pentobarbital anaesthetized dogs (35 mg/kg i.v.).

The compound of the Formula I exhibits significant anti-angina effect (the activity related to that of papaverin amounts to 1.40), it shows an expressed auricle and ventricle antiarrhythmial and antifibrillatory effect respectively (Table 1) and acts advantageously on several parameters of the function of the cardiovascular system too (Table 1).

Thus among others the compound of the present invention increases the blood-flow of peripherial blood-vessels (arteria femoralis, carotis, vertebralis), decreases the resistance of the said blood-vessels and improves the efficiency of cardial function. Due to the antiangina activity it increases coronary flow, decreases coronary resistance and the oxygen consumption of the left ventricle, increases the myocardial oxygen supply per oxygen consumption quotient and thus improves the oxygenization of the cardial muscle to a significant extent. The compound of the Formula I is a potent drug in the treatment of cardiovascular diseases, particularly Angina pectoris, auricle, and ventricle arrhythmia and peripherial circulatory disorders caused by reduced blood flow.

The structurally similar 6,7-diethoxy-1-(3-ethoxy-4-hydroxy-benzyl)-3,4-dihydro-isoquinoline of the Formula VII

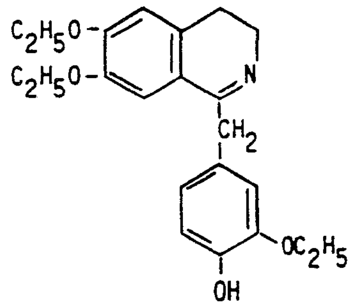

(VII)

is described in prior art as a metabolite of Drotaverin. This compound also exhibits an anti-angina effect (its activity relates to that of papaverin amounts to 1.28) and accordingly increases coronary flow, decreases coronary resistance and the oxygen consumption of the left ventricle, it increases the myocardial oxygen supply per oxygen consumption quotient and thereby improves the oxigenization of the cardial muscle (Table 2). The compound of the Formula VII rises the fibrillation threshold of the auricle and ventricle too and also exhibits anti-arrhythmial activity (Table 2).

Contrary to the compound of the Formula I of the present invention, the compound of the Formula VII decreases the blood flow of peripherial blood-vessels (Arteria femoralis, carotis, vertebralis) and at the same time increases the resistance of the said blood-vessels, thus it can not be used for the treatment of peripherial circulatory disorders or for the improvement of the blood supply of the limbs or of the brain.

The acute toxicity of the compound of the Formula I is characterized by the data enumerated in Table 3.

TABLE 1

Summary of the tested main circulatory-pharmacological effects of the 6,7-diethoxy-1-(4-ethoxy-3-hydroxy-benzyl)-3,4-dihydro-isoquinoline of the Formula I on anaesthetized dogs and cats (average + S.E.) (Dose: 2 mg/kg i.v.)

| Parameter | n | Basic value | Changed value | Percental difference | Duration of effect (min) |
|---|---|---|---|---|---|
| Arterial blood pressure (Hgmm) (on dogs) | 12 | 121 ± 5 | 94 ± 4 | −22 | 5 |
| Heart frequence/min (on dogs) | 10 | 146 ± 8 | 114 ± 9 | −22 | 31 |
| Contractibility of the left ventricle (dP/dt, Hgmm/sec, on dogs) | 6 | 5105 ± 434 | 4248 ± 865 | −17 | 2 |
| Minute volume (ml/min) (on dogs) | 6 | 875 ± 42 | 975 ± 34 | +11 | 4 |
| Frequence volume/ml/ (on dogs) | 6 | 5.5 ± 0.3 | 8.8 ± 0.9 | +60 | 35 |
| Total peripherial resistance (dyn. sec. $cm^{-5}$ on dogs) | 6 | 10652 ± 991 | 8185 ± 748 | −23 | 5 |
| Function of the left ventricle (mkg/min, on dogs) | 6 | 1.24 ± 0.08 | 0.99 ± 0.16 | −20 | 7 |
| Coronary flow (ml/min/100 g, on dogs) | 6 | 95 ± 5 | 129 ± 9 | +36 | 16 |
| Coronary resistance (Hgmm/ml/min/100 g, on dogs) | 7 | 1.16 ± 0.06 | 0.76 ± 0.06 | −34 | 15 |
| Oxygen consumption of the left ventricle (ml/min/100 g, on dogs) | 6 | 11.6 ± 0.9 | 8.2 ± 1.2 | −29 | 22 |
| Efficiency of cardial function (mkg/ml/100 g, on dogs) | 6 | 0.11 ± 0.02 | 0.16 ± 0.02 | +45 | 6 |
| Myocardial oxygenization ($O_2$ supply per $O_2$ consumption, on dogs) | 6 | 1.70 ± 0.07 | 2.95 ± 0.21 | +74 | 30 |
| A. femoralis flow (ml/min) | 6 | 34 ± 7 | 49 ± 10 | +44 | 3 |
| A. femoralis resistance (Hgmm/ml/min, on dogs) | 6 | 4.84 ± 0.86 | 2.66 ± 0.42 | −45 | 3 |
| A. carotis flow (ml/min, on dogs) | 6 | 70 ± 7 | 89 ± 13 | +27 | 7 |
| A. carotis resistance (Hgmm/ml/min, on dogs) | 6 | 2.33 ± 0.23 | 2.02 ± 0.32 | −13 | 7 |
| A. vertebralis flow (Hgmm/ml/min, on dogs) | 6 | 43 ± 6 | 65 ± 9 | +51 | 2 |
| A. vertebralis resistance (Hgmm/ml/min, on dogs) | 6 | 3.28 ± 0.33 | 1.81 ± 0.17 | −45 | 2 |

TABLE 1 (continued)

| Parameter | n | Basic value | Changed value | Percental difference | Duration of effect (min) |
|---|---|---|---|---|---|
| Auricle fibrillation threshold (mA, in cats) | 6 | 0.73 ± 0.10 | 1.15 ± 0.11 | +58 | 11 |
| Ventricle fibrillation threshold (mA, on cats) | 6 | 2.07 ± 0.10 | 2.58 ± 0.09 | +25 | 13 |
| Arterial blood pressure (Hgmm, on cats) | 6 | 139 ± 5 | 79 ± 8 | −43 | 9 |

TABLE 2

Summary of the tested main circulatory-pharmacological effects of the 6,7-diethoxy-1-(3-ethoxy-4-hydroxy-benzyl)-3,4-dihydro-isoquinoline of the Formula VII on anaesthetized dogs and cats (average + S.E.) (Dose: 1 mg/kg i.v.)

| Parameter | n | Basic value | Changed value | Percental difference | Duration of effect (min) |
|---|---|---|---|---|---|
| Arterial blood pressure (Hgmm, on dogs) | 6 | 147 ± 9 | 115 ± 11 | −22 | 8 |
| Heart frequence/min (on dogs) | 6 | 173 ± 8 | 160 ± 9 | −8 | 7 |
| Contractibility of the left ventricle (dp/dt, Hgmm/sec, on dogs) | 6 | 5625 ± 557 | 4883 ± 419 | −13 | −13 |
| Minute volume (ml/min, on dogs) | 5 | 1500 ± 153 | 1466 ± 233 | +2 | 3 |
| Frequence volume (ml) (on dogs) | 5 | 8.8 ± 1 | 9.5 ± 1.2 | +8 | 5 |
| Total peripherial resistance (dyn. sec. $cm^{-5}$, on dogs) | 5 | 7015 ± 238 | 4785 ± 275 | −32 | 14 |
| Function of the left ventricle (mkg/min, on dogs) | 5 | 2.74 ± 0.49 | 2.22 ± 0.56 | −19 | 13 |
| Coronary flow (ml/min/100 g, on dogs) | 5 | 73 ± 13 | 86 ± 13 | +18 | 15 |
| Coronary resistance (Hgmm/ml/min/100 g, on dogs) | 5 | 1.03 ± 0.10 | 1.42 ± 0.22 | −22 | 11 |
| Oxygen consumption of the left ventricle (ml/min/100 g, on dogs) | 5 | 8.4 ± 0.9 | 7.9 ± 1.9 | −6 | 9 |
| Efficiency of cardial function (mkg/ml/100 g on dogs) | 5 | 0.32 ± 0.03 | 0.28 ± 0.05 | −13 | 20 |
| Myocardial oxygenization ($O_2$ supply per $O_2$ consumption, on dogs) | 5 | 1.62 ± 0.06 | 1.79 ± 0.10 | +10 | 18 |
| A. femoralis flow (ml/min) | 5 | 46 ± 5 | 42 ± 5 | −9 | 11 |

TABLE 2 (continued)

| Parameter | n | Basic value | Changed value | Percental difference | Duration of effect (min) |
|---|---|---|---|---|---|
| A. femoralis resistance (Hgmm/ml/min, on dogs) | 5 | 3.48 ± 0.45 | 3.69 ± 0.68 | +6 | 18 |
| A. carotis flow (ml/min, on dogs) | 5 | 91 ± 9 | 80 ± 10 | −12 | 12 |
| A. carotis resistance (Hgmm/ml/min, on dogs) | 5 | 2.04 ± 0.3 | 2.19 ± 0.4 | +7 | 15 |
| A. vertebralis flow (Hgmm/ml/min, on dogs) | 5 | 54 ± 9 | 50 ± 7 | −7 | 7 |
| A. vertebralis resistance (Hgmm/ml/min, on dogs) | 5 | 3.22 ± 0.61 | 3.65 ± 0.32 | +12 | 12 |
| Auricle fibrillation threshold (mA, on cats) | 5 | 0.52 ± 0.07 | 0.77 ± 0.19 | +48 | 9 |
| Ventricle fibrillation threshold (mA, on cats) | 5 | 0.70 ± 0.10 | 1.00 ± 0.18 | +43 | 12 |
| Arterial blood pressure (Hgmm, on cats) | 5 | 142 ± 11 | 110 ± 16 | −23 | 5 |

TABLE 3

Acute Toxicity Data of the 6,7-Diethoxy-1-(4-Ethoxy-3-Hydroxy-Benzyl)-3,4-Dihydro-Isoquinoline of the Formula I

$LD_{50}$ = 24.92 mg/kg i.v. on rats ♀
= 22.98 mg/kg i.v. on rats ♂

$LD_{50}$ = 269.18 mg/kg p.o. on mice ♂
= 164.99 mg/kg p.o. on mice ♀

$LD_{50}$ = 493 mg/kg p.o. on rats ♂
= 625 mg/kg p.o. on rats ♀

According to a further feature of the present invention there are provided pharmaceutical compositions comprising as active ingredient the compound of the Formula I or a physiologically acceptable salt thereof in admixture with suitable inert non-toxical solid or liquid carriers or diluents.

The pharmaceutical compositions may be used either in solid (e.g. tablet, capsule, dragée) or liquid (e.g. solution, suspension, emulsion) form.

The active ingredient content of the pharmaceutical compositions may vary between wide ranges and may be from about 0.001 to about 95%. The daily active ingredient dosage may also vary between wide ranges and depends on the various circumstances of the given case (e.g. seriousness of the state of the patient, age and body weight of the patient, the formulation used, the activity of the active ingredient etc.).

The pharmaceutical compositions of the present invention can be prepared by methods of pharmaceutical industry known per se.

Further details of the present invention are to be found in the Examples without limiting our invention to the said Examples.

Example 1

20.0 g (66.6 mmoles) of methyl-(3-benzyloxy-4-ethoxy-phenyl)-acetate and 100 ml of a 10% by weight sodium hydroxide solution are stirred at 110°C for 2 hours. After the solid substance has been completely dissolved the solution is cooled in an ice-water bath to +10°C and acidified with concentrated hydrochloric acid to the pH value of 1. The precipitated flocky white crystals are filtered off, washed with distilled water and dried in a vacuo desiccator. The crude product is recrystallized from a mixture of benzene and petrolether. Thus 17.9 g of 3-benzyloxy-4-ethoxy-phenyl-acetic acid are obtained, yield: 93.8%. Mp.: 95—97°C.

Analysis: for the Formula $C_{17}H_{18}O_4$

| | | |
|---|---|---|
| calculated: | C 71.31% | H 6.33%; |
| found: | C 71.18% | H 6.11%. |

IR (in a potassium bromide capsule): 1712 $cm^{-1}$ (>C=O)

NMR: (solvent $CDCl_3$): 1.38 (t, 3H, $CH_3$—; 3.44 (s, 2H, —$CH_2$—COO—; 3.77—4.26 (q, 2H, —$CH_2$—; 5.03/s, 2H, benzyl-$CH_2$; 6.47—7.57/m 8H, aromatic H; 9.86/s, 1H, —COOH.

Example 2

6.30 g (22.0 mmoles) of 3-benzyloxy-4-ethoxy-phenyl-acetic acid are dissolved in 60 ml of xylene. 4.80 g (22.9 mmoles) of 3,4-diethoxy-β-phenyl-ethyl amine and 1 g of Varion KS cation exchanging resin (previously washed with 5N hydrochloric acid and distilled water) are added. The reaction mixture is heated to boiling under a water-separator until an equivalent amount of water is removed. The hot solution is filtered, the filtrate is cooled and allowed to crystallize. The precipitated crystalline substance is filtered off, washed with petrolether and dried. Thus 8.20 g of N-(3-benzyloxy-4-ethoxy-phenyl-acetyl)-3,4-diethoxy-β-phenyl-ethyl-amide are obtained, yield: 78.0%, mp.: 123—124°C.

Analysis for the Formula $C_{29}H_{55}NO_5$:

| | | | |
|---|---|---|---|
| calculated: | C 72.92% | H 7.38% | N 2.93%; |
| found: | C 72.86% | H 7.59% | N 2.89%. |

IR (in a potassium bromide pastille): $1641^{-1}$ (C=O)

NMR (solvent $CDCl_3$): 1.39 and 1.44 (t, 9H, $CH_3$—); 2.59 (t, 2H, Ar—$\underline{CH_2}$—$CH_2$—); 3.08—3.68 (m, 4H, —$CH_2$—C=O); —$CH_2$—N=C—O); 3.78—4.37 (m, 6H, $CH_3$—$CH_2$—O—); 5.04 (s, 2H, benzyl-$CH_2$—);, 5.40 (s, 1H, —NH—); 6.28—7.58 (m, 11H, aromatic H).

Example 3

10.0 g (20.9 mmoles) of N-(3-benzyloxy-4-ethoxy-phenyl-acetyl)-3,4-diethoxy-β-phenyl-ethyl-amide are dissolved in 60 ml of anhydrous chloroform. The solution is cooled to 0°C, whereupon a suspension of 5.60 g (26.8 mmoles) of phosphorous pentachloride and 60 ml of anhydrous chloroform is added at 0°C. The mixture thus obtained is allowed to stand at 0°C for 30 minutes and at room temperature for 2 days. The reaction mixture is diluted with anhydrous ether. The precipitated crystalline substance is filtered off washed with anhydrous ether and dried. Thus 9.65 g of 1-(3-benzyloxy-4-ethoxy-benzyl)-6,7-diethoxy-3,4-dihydro-isoquinoline-dihydrochloride are obtained, yield: 92.9%, mp.: 209—210°C (decomposition).

Analysis: for the Formula $C_{29}H_{34}ClNO_4$:

| | | | |
|---|---|---|---|
| calculated: | C 70.21% | H 6.90% | N 2.82%; |
| found: | C 69.80% | H 6.70% | N 3.25%. |

IR (in a potassium bromide pastille): 1670 $cm^{-1}$ (>C=$N^{\oplus}$H—).

Example 4

3.50 g (7.05 mmoles) of 1-(3-benzyloxy-4-ethoxy-benzyl)-6,7-diethoxy-3,4-dihydro-isoquinoline-hydrochloride are dissolved in 100 ml of methanol and the solution is poured into a pre-hydrogenated suspension of 1.50 g of a 10% palladium/charcoal catalyst and 20 ml of methanol. The hydrogenation is carried out at atmospheric pressure and room temperature. The calculated amount of hydrogen (169 ml) is taken up within about 10 minutes. Hydrogenation is then stopped, the catalyst is filtered off, and the solvent is distilled off in vacuo. The residual solid crude product (2.80 g) is crystallized from a mixture of methanol and ether. Thus 2.00 g of 6.7-diethoxy-1-(4-ethoxy-3-hydroxy-benzyl)-6,7-dihydro-isoquinoline-hydrochloride are obtained, yield: 69.8%, mp.: 223—225°C.

Analysis for the Formula $C_{22}H_{28}ClNO_4$:

| | | | |
|---|---|---|---|
| calculated: | C 65.09% | H 6.95% | N 3.45%; |
| found: | C 64.97% | H 6.99% | N 3.66%. |

IR (potassium bromide capsule): 3300 $cm^{-1}$ (—OH);
1622 $cm^{-1}$ (>C=$N^{\oplus}$H—).

Example 5

30 mg tablets are prepared (batch-size 10.000 tablets). The following components are used:

| Component | Amount, g |
| --- | --- |
| a) Compound of the Formula I | 300 |
| (b) Potato starch | 530 |
| c) Lactose | 600 |
| d) Gelatine | 18 |
| e) Distilled water | 100 |
| f) Ethanol (96 vol.%) | 200 |
| g) Sodium pyrosulfite | 2 |
| h) Talc | 30 |
| i) Stearic acid | 20 |

The homogeneous powder mixture of components a), b) and c) is wetted with a solution of components d), e), f) and g) in a suitable kneading machine in small portions, whereupon it is granuled on a sieve (mash size 0.3 mm) and dried at 40°C. The dry granules are re-granulated on a sieve (mash size 0.15 mm), thoroughly admixed with the homogenous powder mixture of components h) and i) and pressed into tablets weighing 150 mg.

Example 6

100 mg retard tablets and dragées are prepared (batch size 10.000).
The following components are used:

| Component | Amount, g |
| --- | --- |
| a) Compound of the Formula I | 1000 |
| b) Crystalline cellulose | 450 |
| c) Lactose | 765 |
| d) Copolymer of vinyl pyrrolidone and vinyl acetate | 180 |
| e) Distilled water | 150 |
| f) Ethanol (96 vol.%) | 350 |
| g) Sodium pyrosulfite | 5 |
| h) Colloidal silicic acid | 24 |
| i) Magnesium stearate | 25 |
| j) Stearine | 50 |

The homogeneous powder mixture of components a), b) and c) is granulated and dried with a suspension of components d), e), f), g) and j) in an apparatus under overpressure at 40°C according to the principle of fluidisation. The dry granules are regranulated on a sieve (mash size 0.15 mm), admixed with a homogeneous powder mixture of components h) and i) and pressed into tablets and capsules weighing 250 mg respectively.

The active ingredient delivery of the tablets should reach 80% within 6—11 hours according to the so-called "half-change" method. The dragée core is coated with a sugar layer by methods known per se.

Example 7

Injections containing 20 mg/2 $cm^3$ are prepared (batch size 10.000). The following components are used:

| Component | Amount |
|---|---|
| a) Compound of the Formula I | 100 g |
| b) Distilled water ad | 10.000 ml |
| c) Sodium pyrosulfite | 15 g |
| d) Sodium chloride | 27 g |

The solution of components b), c) and d) is prepared under carbon dioxide and the exclusion of air, whereupon component a) is dissolved and the pH of the solution is adjusted to 3.7—4.2 with the aid of 1*N* hydrochloric or 1*N* sodium hydroxide, if necessary. The solution is diluted to 10,000 ml and thereafter aseptically filtered sterile through a bacterium filter and filled into 2.2 ml ampoules in a known manner. The solution is filled into brown glass ampoules.

**Claims**

1. 6,7-Diethoxy-1-(4-ethoxy-3-hydroxy-benzyl)-3,4-dihydro-isoquinoline of the Formula I

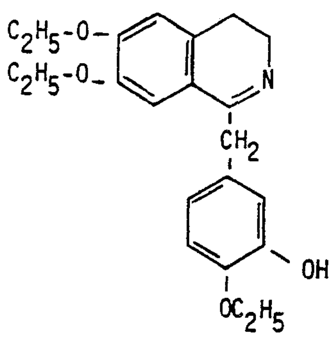

(I)

and physiologically acceptable salts thereof.

2. Process for the preparation of 6,7-diethoxy-1-(4-ethoxy-3-hydroxy-benzyl)-3,4-dihydro-isoquinoline of the Formula I

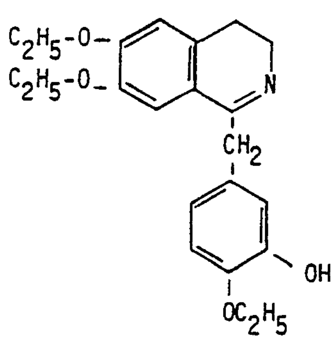

(I)

and physiologically acceptable salts thereof which comprises hydrolysing a compound of the general Formula VI

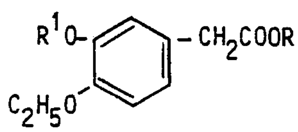

(VI)

(wherein $R^1$ is a group removable by hydrogenolysis, preferably a benzyl group and R is an alkyl group having 1—6 carbon atoms), reacting the compound of the general Formula V

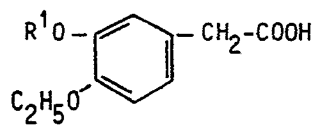

(V),

thus obtained (wherein $R^1$ is as stated above) with an amine of the Formula IV

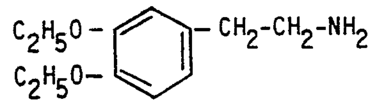

(IV)

or a salt thereof in the presence of a catalyst, subjecting the amide of the general Formula III

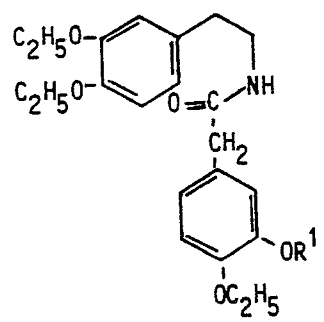

(III) ,

thus obtained (wherein $R^1$ is as stated above) to ring-closure and subjecting the compound of the general Formula II

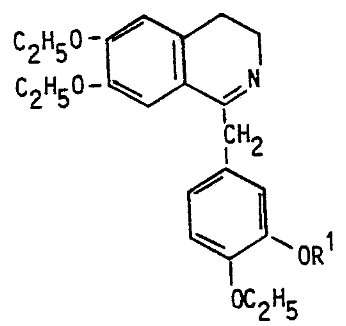

(II) .

(wherein $R^1$ is as stated above) or a salt thereof thus obtained to hydrogenolysis and if desired converting the compound of the Formula I thus obtained into a physiologically acceptable salt thereof or setting free the same from its salt.

3. Process according to Claim 2 which comprises hydrolysing a compound of the general Formula VI (wherein R and $R^1$ are as stated in Claim 1) in alkaline medium.

4. Process according to Claim 2 which comprises using a catalyst capable of delivering protons, preferably a mineral acid, sulfonic acid or cation-exchanging resin.

5. Process according to Claim 2 which comprises carrying out the reaction of the compounds of the general Formulae V and IV (wherein $R^1$ is as stated in Claim 2) in an organic solvent, preferably in an aprotic solvent as medium at the boiling point of the solvent.

6. Process according to Claim 2 which comprises carrying out ring-closure in the absence or presence of a solvent, preferably in chloroform, benzene or acetonitrile, in the presence of phosphorous pentachloride, phosphoryl chloride, thionyl chloride or poly phosphoric acid or a mixture thereof.

7. Process according to Claim 2 which comprises isolating the compound of the general Formula II (wherein $R^1$ is as stated in Claim 2) by making the reaction mixture alkaline and extracting with a solvent.

8. Process according to Claim 2 which comprises hydrogenating a compound of the general Formula II (wherein $R^1$ is as stated in Claim 2) in the presence of a palladium/charcoal, platinum or Raney-nickel catalyst, preferably in an aliphatic alcohol having 1—4 carbon atoms, at atmospheric pressure and room temperature.

9. Process according to Claim 2 which comprises converting the compound of the Formula I into a neutral or acidic salt comprising a physiologically acceptable anion, preferably into the hydrochloride, hydrobromide, sulfate, hydrogen sulfate, phosphate, hydrogen phosphate, succinate, citrate, maleate, salicylate or benzoate.

10. Pharmaceutical composition comprising as active ingredient the compound of the Formula I or a physiologically acceptable salt thereof and an inert non-toxic carrier.

11. Process for the preparation of pharmaceutical compositions according to Claim 10 which comprises admixing the compound of the Formula I or a physiologically acceptable salt thereof with inert non-toxic carriers.

**Patentansprüche**

1. 6,7-Diäthoxy-1-(4-äthoxy-3-hydroxy-benzyl)-3,4-dihydroisochinolin der Formel I

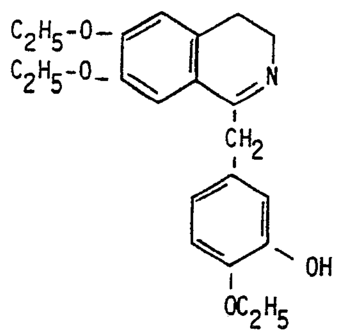

(I)

und dessen physiologisch verträgliche Salze.

2. Verfahren zur Herstellung von 6,7-diäthoxy-1-(4-äthoxy-3-hydroxy-benzyl)-3,4-dihydro-isochinolin der Formel I

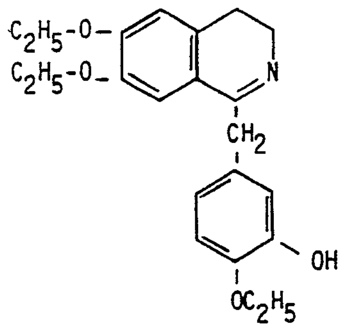

(I)

und dessen physiologisch verträglichen Salzen, dadurch gekennzeichnet, daß man eine Verbindung der allgemeinen Formel VI

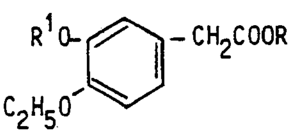

(VI)

worin

$R^1$ eine durch Hydrogenolyse abspaltbare Gruppe, vorzugsweise eine Benzylgruppe, und

R eine $C_{1-6}$-Alkylgruppe bedeuten,

hydrolysiert, die so erhaltene Verbindung der allgemeinen Formel V

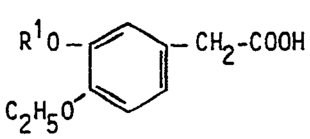

(V)

worin $R^1$ die oben angegebene Bedeutung hat, mit einem Amin der Formel IV

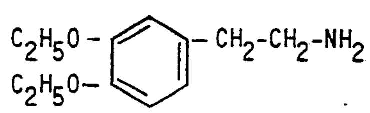

(IV)

oder einem seiner Salze in Gegenwart eines Katalysators umsetzt, das so erhaltene Amid der allgemeinen Formel III

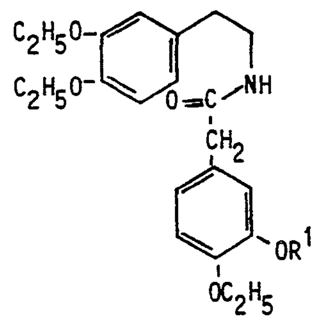

(III)

worin $R^1$ die oben angegebene Bedeutung hat, dem Ringschluß unterwirft, und die so erhaltene Verbindung der allgemeinen Formel II

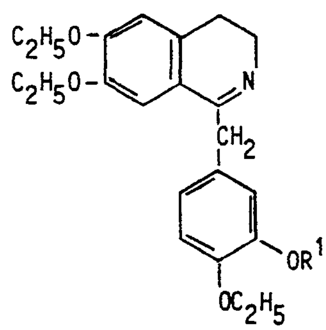

(II) ,

worin $R^1$ die oben angegebene Bedeutung hat, oder eines seiner Salze zur Verbindung der Formel I hydrogenolysiert, die gewünschtenfalls in eines ihrer physiologisch verträglichen Salze übergeführt oder aus einem Salz freigesetzt werden kann.

3. Verfahren nach Anspruch 2, dadurch gekennzeichnet, daß man die Hydrolyse einer Verbindung der allgemeinen Formel VI, worin R und $R^1$ die oben angegebene Bedeutung haben, in alkalischem Medium durchführt.

4. Verfahren nach Anspruch 2, dadurch gekennzeichnet, daß man einen Protonen-liefernden Katalysator, vorzugsweise eine Mineralsäure, Sulfonsäure oder ein Kationenaustauscherharz, verwendet.

5. Verfahren nach Anspruch 2, dadurch gekennzeichnet, daß man die Umsetzung der Verbindungen der allgemeinen Formel V mit der Verbindung der Formel IV, worin $R^1$ die oben angegebene Bedeutung hat, in einem organischen Lösungsmittel, vorzugsweise in einem aprotischen Lösungsmittel, als Medium beim Siedepunkt des Lösungsmittels durchführt.

6. Verfahren nach Anspruch 2, dadurch gekennzeichnet, daß man den Ringschluß in Ab- oder in Anwesenheit eines Lösungsmittels, vorzugsweise Chloroform, Benzol oder Acetonitril, in Gegenwart von Phosphorpentachlorid, Phosphorylchlorid, Thionylchlorid und/oder Polyphosphorsäure durchführt.

7. Verfahren nach Anspruch 2, dadurch gekennzeichnet, daß man zur Isolierung der Verbindung der allgemeinen Formel II, worin $R^1$ die oben angegebene Bedeutung hat, die Reaktionsmischung alkalisch macht und mit einem Lösungsmittel extrahiert.

8. Verfahren nach Anspruch 2, dadurch gekennzeichnet, daß man die Hydrierung einer Verbindung der allgemeinen Formel II, worin $R^1$ die oben angegebene Bedeutung hat, in Gegenwart eines Palladium-Tierkohle-, Platin- oder Raneynickel-Katalysators, vorzugsweise in einem aliphatischen Alkohol mit 1—4 C-Atomen, bei Atmosphärendruck und Raumtemperatur durchführt.

9. Verfahren nach Anspruch 2, dadurch gekennzeichnet, daß man die Verbindung der Formel I in ein neutrales oder saures Salz mit einem physiologisch verträglichen Anion, vorzugsweise in das Hydrochlorid, Hydrobromid, Sulfat, Hydrogensulfat, Phosphat, Hydrogenphosphat, Succinat, Citrat, Maleat, Salicylat oder Benzoat überführt.

10. Pharmazeutische Komposition, die als Wirkstoff die Verbindung der Formel I oder eines ihrer physiologisch verträglichen Salze und einen inerten nicht-toxischen Träger enthält.

11. Verfahren zur Herstellung von pharmazeutischen Kompositionen gemäß Anspruch 10, dadurch gekennzeichnet, daß man die Verbindung der Formel I oder eines ihrer physiologisch verträglichen Salze mit inerten nicht-toxischen Trägern vermischt.

**Revendications**

1. 6,7-diéthoxy-1-(4-éthoxy-3-hydroxy-benzyl)-3,4-dihydro-isoquinoléine répondant à la formule I:

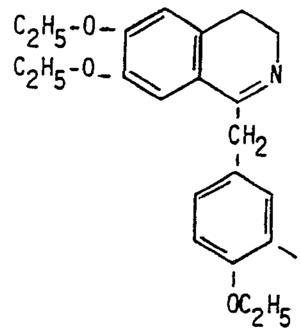

(I)

et leurs sels physiologiquement acceptables.

2. Procédé de préparation de 6,7-diéthoxy-1-(4-éthoxy-3-hydroxy-benzyl)-3,4-dihydro-isoquinoléine de formule I

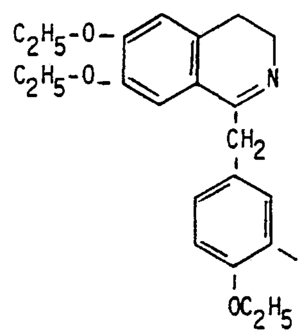

(I)

et de leurs sels physiologiquement acceptables, qui comprend le fait d'hydrolyser un composé de formule générale VI:

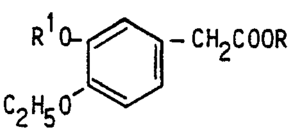

(VI),

(dans laquelle $R^1$ est un groupe amovible par hydrogénolyse, de préférence un radical benzyle et R est un radical alkyle de 1 à 6 atomes de carbone), de faire réagir le composé de formule générale V:

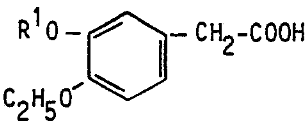

(V),

ainsi obtenu (dans laquelle $R^1$ est tel qu'indiqué plus haut) avec une amine de formule IV

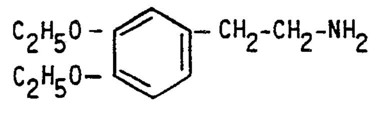

(IV)

ou un sel de celle-ci en présence d'un catalyseur, de soumettre l'amide de formule générale III

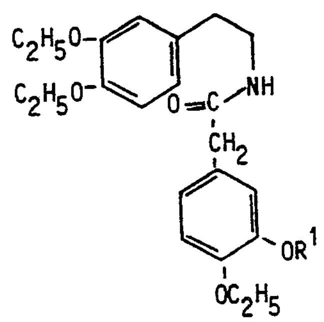

(III) ,

ainsi obtenu (dans laquelle $R^1$ est tel qu'indiqué plus haut) à une condensation du noyau et de soumettre le composé de formule générale II:

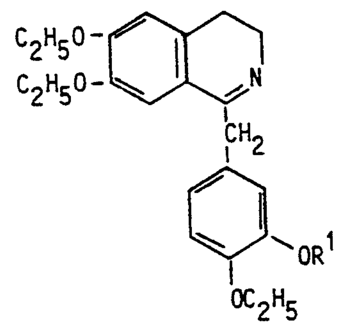

(II) .

(dans laquelle $R^1$ est tel que ci-dessus) ou un sel de celui-ci ainsi obtenu à une hydrogénolyse et éventuellement de convertir le composé de formule I ainsi obtenu en un sel physiologiquement acceptable de celui-ci ou de le libérer de son sel.

3. Procédé selon la revendication 2, qui comprend le fait d'hydrolyser un composé de formule générale VI (dans laquelle R et $R^1$ sont tels qu'indiqués dans la revendication 1) dans un milieu alcalin.

4. Procédé selon la revendication 2, qui comprend le fait d'utiliser un catalyseur capable de dégager des protons, de préférence un acide minéral, un acide sulfonique ou une résine échangeuse de cations.

5. Procédé selon la revendication 2, qui comprend le fait d'effectuer la réaction des composés de formules générales V et IV (dans lesquelles $R^1$ est tel qu'indiqué dans la revendication 2) dans un solvant organique, de préférence dans un solvant aprotique comme milieu, au point d'ébullition du solvant.

6. Procédé selon la revendication 2, qui comprend le fait d'effectuer la condensation du noyau en l'absence ou en présence d'un solvant de préférence dans le chloroforme, le benzène ou l'acétonitrile, en présence de pentachlorure phosphoreux, de chlorure de phosphoryle, de chlorure de thionyle, ou d'un acide polyphosphorique ou d'un mélange de ceux-ci.

7. Procédé selon la revendication 2, qui comprend le fait d'isoler le composé de formule générale II (dans laquelle $R^1$ est tel que dans la revendication 2) en alcalinisant le mélange de réaction et en l'extrayant avec un solvant.

8. Procédé selon la revendication 2, qui comprend le fait d'hydrogéner un composé de formule générale II (dans laquelle $R^1$ est tel que dans la revendication 2) en présence d'un charbon palladié, d'un catalyseur au platine ou au nickel Raney, de préférence dans un alcool aliphatique contenant 1 à 4 atomes de carbone, sous pression atmosphérique et à la température ambiante.

9. Procédé selon la revendication 2, qui comprend le fait de convertir le composé de formule I en un sel neutre ou acide comprenant un anion physiologiquement acceptable, de préférence dans un chlorhydrate, bromhydrate, sulfate, hydrogéno-sulfate, phosphate, hydrogéno-phosphate, succinate, citrate, maléate, salicylate ou benzoate.

10. Composition pharmaceutique, qui comprend, à titre d'ingrédient actif, le composé de formule I ou un sel physiologiquement acceptable de celui-ci et un véhicule inerte non toxique.

11. Procédé de préparation de compositions pharmaceutiques selon la revendication 10, qui comprend le fait de mélanger le composé de formule I ou un sel physiologiquement acceptable de celui-ci avec des véhicules inertes non toxiques.